(19) Europäisches Patentamt

European Patent Office

Office européen des brevets

(11) Veröffentlichungsnummer : **0 072 902**
**B1**

(12) **EUROPÄISCHE PATENTSCHRIFT**

(45) Veröffentlichungstag der Patentschrift :
24.04.85

(21) Anmeldenummer : 82105693.4

(22) Anmeldetag : 26.06.82

(51) Int. Cl.⁴ : **G 01 N 33/574**, G 01 N 33/543,
C 12 Q 1/00

(54) **Verfahren zur Bestimmung von carcinoembryonalem Antigen (CEA) und zur Bestimmung geeignete Antikörperlösung.**

(30) Priorität : 21.08.81 CH 5403/81
11.11.81 CH 7258/81

(43) Veröffentlichungstag der Anmeldung :
02.03.83 Patentblatt 83/09

(45) Bekanntmachung des Hinweises auf die Patenterteilung : 24.04.85 Patentblatt 85/17

(84) Benannte Vertragsstaaten :
AT BE CH DE FR GB IT LI NL SE

(56) Entgegenhaltungen :
EP-A- 0 005 271
DE-A- 2 755 008
US-A- 4 180 556
CLINICAL CHEMISTRY, Band 26, Nr. 12 1980 R. MAIOLINI et al. "Study of an Enzyme Immunoassay Kit for Carcinoembryonic Antigen" Seiten 1718 bis 1722

(73) Patentinhaber : **F. HOFFMANN-LA ROCHE & CO. Aktiengesellschaft**
**CH-4002 Basel (CH)**

(72) Erfinder : **Gallati, Harald, Dr.**
**Ingelsteinweg 13**
**CH-4143 Dornach (CH)**
Erfinder : **Brodbeck, Hans**
**Lindenstrasse 12**
**CH-4142 Münchenstein (CH)**

(74) Vertreter : **Kloter, Rudolf et al**
**Grenzacherstrasse 124 Postfach 601**
**CH-4002 Basel (CH)**

# 0 072 902

**Beschreibung**

Aus Clin. Chem. 26/12, 1718-1722 (180) ist ein Enzymimmunverfahren zur Bestimmung von carcinoembryonalem Antigen (CEA) bekannt. Bei diesem Verfahren werden vorbehandelte Serum- oder Plasmaproben in einer ersten Stufe während zwei Stunden bei 45 °C mit Kugeln inkubiert, die mit Meerschweinchen-anti-CEA sensibilisiert sind. Nach einem Waschschritt werden die Kugeln während zwei Stunden bei 45 °C mit einem Konjugat aus Ziegen-anti-CEA und Meerrettich-Peroxidase inkubiert. Nach Entfernen von überschüssigem Konjugat durch Waschen wird die Enzymaktivität an der Festphase nach bekannten Methoden bestimmt. Durch Vergleich dieses Wertes mit einer in analoger Weise hergestellten Standardkurve wird der Gehalt an CEA in der Probe ermittelt.

Im Rahmen der vorliegenden Erfindung wurde nun überraschenderweise gefunden, dass man dieses Verfahren ohne Verlust an Sensitivität in der gleichen Zeit auch ohne den zeitaufwendigen und mühsamen Waschschritt zwischen der ersten und der zweiten immunologischen Reaktion durchführen kann. Diese Vereinfachung wird ermöglicht, wenn man die zu untersuchende Probe mit einem ersten CEA-Antikörper, der an den wasserunlöslichen Träger gebunden ist, und den zweiten CEA-Antikörper, an den Peroxidase gebunden ist, in Gegenwart von 0,4-1,0 mol/l Phosphationen, von 0,2-0,4 mol/l Sulfationen oder von 0,2-0,4 mol/l Tartrationen durchführt.

Im weiteren hat sich gezeigt, dass durch die Verwendung der erwähnten Ionen auch die zweistufige Methode gemäss Clin. Chem. (loc. cit.) zeitlich verkürzt werden kann, indem die zweite Inkubation ohne Sensitivitätsverlust in der halben Zeit, d. h. also in einer Stunde durchgeführt werden kann.

Das vorerwähnte Enzymimmunverfahren zur Bestimmung von Antigenen, im vorliegenden Fall von CEA, ist allgemein als sogenanntes Festphasen-Sandwichverfahren bekannt. Bei diesem Verfahren kann das Enzymm, im speziellen Fall die Peroxidase, direkt oder über eine Biotin/Avidin-Brücke an den Antikörper gebunden sein.

Die vorliegende Erfindung betrifft somit ein Festphasen-Sandwichverfahren zur raschen Bestimmung von carcinoembryonalem Antigen (CEA) durch Inkubation der zu untersuchenden Probe mit einem ersten CEA-Antikörper, der an einem wasserunlöslichen Träger gebunden ist oder wird, und einem CEA-Antikörper, an den Peroxidase direkt oder über eine Biotin/Avidin-Brücke gebunden ist, Trennen von fester und flüssiger Phase, Messen der Peroxidaseaktivität, entweder in der festen oder flüssigen Phase als Ausmass des vorhandenen CEA, welches dadurch gekennzeichnet ist, dass die Inkubation in Gegenwart von 0,4-1,0 mol/l Phosphationen, von 0,2-0,4 mol/l Sulfationen oder von 0,2-0,4 mol/l Tartrationen durchgeführt wird.

Wie bereits erwähnt, kann nach dem erfindungsgemässen Festphasen-Sandwichverfahren die zu untersuchende Probe zuerst mit dem einen CEA-Antikörper, der an einen wasserunlöslichen Träger gebunden ist, inkubiert werden, und anschliessend nach einem Waschschritt mit dem zweiten CEA-Antikörper, der mit Peroxidase markiert ist, inkubiert werden. Da die aktivierende Wirkung von 0,4-1,0 mol/l Phosphationen, von 0,2-0,4 mol/l Sulfationen oder von 0,2-0,4 mol/l Tartrationen auf die zweite Inkubation einwirkt, wird demgemäss diese zweite Inkubation in Gegenwart dieser Ionen durchgeführt.

Wie bereits früher erwähnt, kann als erster CEA-Antikörper, der an einen wasserunlöslichen Träger gebunden ist, ein Meerschweinchen-CEA-Antikörper verwendet werden. Als zweiter CEA-Antikörper, an den Peroxidase gebunden ist, kann Ziegen-anti-CEA verwendet werden.

Als erster und zweiter CEA-Antikörper können aber auch zwei verschiedene monoklonale Maus-CEA-Antikörper verwendet werden, die beide gegen CEA, aber unterschiedliche Epitope von CEA, gerichtet sind.

Im weiteren ist es auch möglich, als ersten Antikörper einen monoklonalen Maus-CEA-Antikörper und als zweiten einen polyklonalen Antikörper aus einem Antiserum eines Tieres, z. B. der Ziege zu verwenden.

Die Peroxidase kann nach an sich bekannten Methoden, z. B. der Methode von Wilson und Nakane in « Immunfluorescence and Related Staining Techniques », 1978, Seite 215, an den Antikörper gebunden werden. Bei den monoklonalen Maus-CEA-Antikörpern hat sich gezeigt, dass bei diesen bekannten Methoden unter Umständen die immunologischen Eigenschaften dieser Antikörper beeinträchtigt werden. In diesem Falle ist es von Vorteil, den monoklonalen Maus-CEA-Antikörper zu biotinylieren und anschliessend mit einem Avidin-Peroxidase-Komplex umzusetzen. Die Herstellung des Avidin-Peroxidase-Komplexes erfolgt in Analogie zur obenerwähnten Methode von Wilson und Nakane. Bei polyklonalen Antikörpern, d. h. Antikörpern aus Tierseren, ist die direkte Bindung der Peroxidase an den Antikörper bevorzugt.

Als wasserunlösliche Träger für den ersten Antikörper kommen in Betracht : organische und anorganische Polymere [Amylase, Dextrane, native oder modifizierte Cellulose, Polyacrylamid, Agarose, Magnetit, poröses Glaspulver, Polyvinylidenfluorid (Kynar) und Latex], die Innenwand von Testgefässen (Teströhrchen, Titrierplatten oder Küvetten aus Glas oder Kunststoff) sowie die Oberfläche von festen Körpern (Stab, Kugel oder andersgeartete Körper aus Glas oder Kunststoff). Besonders geeignete Träger für die erfindungsgemässe Methode sind Glas- und Kunststoffkugeln.

Der Antikörper kann an den wasserunlöslichen Träger physikalisch (adsorptiv) oder chemisch gebunden sein, oder mit Hilfe eines weiteren Reaktionspartners, der seinerseits an einen Träger gebunden ist, während oder nach der Reaktion gebunden werden.

Die immunologischen Reaktionen erfolgen vorzugsweise bei einer Temperatur zwischen 0° und 55 °C. Normalerweise nimmt die immunologische Reaktionsgeschwindigkeit mit höheren Temperaturen zu, wodurch unter sonst gleichen Testbedingungen das Gleichgewicht schneller erreicht wird.

Die neue Methode gemäss vorliegender Erfindung ist ausserordentlich empfindlich und zeichnet sich insbesondere durch ihre kurze Reaktionszeit aus.

Ein Test-kit für die erfindungsgemässe Methode beinhaltet insbesondere ein Gefäss mit CEA-Antikörpern, an die Peroxidase gekoppelt ist, in einer wässrigen Lösung vom pH 4-9, die 0,4-1,0 mol/l Phosphationen, 0,2-0,4 mol/l Sulfationen oder 0,2-0,4 mol/l Tartrationen enthält.

Die nachfolgenden Beispiele erläutern die Erfindung.

Beispiel 1

Quantitative Bestimmung von CEA in Patientenplasmen mit einem monoklonalen CEA-Antikörper und einem gebräuchlichen CEA-Antikörper (Ziegen)

In die erforderliche Anzahl Teströhrchen (10 × 75 mm) werden je 0,2 ml Testlösung (0,8 mol/l NaH$_2$PO$_4$/Na$_2$HPO$_4$, pH 6,5 mit 2 g/l Rinderserumalbumin, 20 % normales Ziegenserum, 0,2 g/l 4-Amino-antipyrin und 0,2 μg/ml Ziegen-Anti-CEA-Peroxidase-Konjugat) pipettiert, 0,050 ml der zu analysierenden Patientenplasmen resp. der CEA-Standards (0 ng/ml CEA, 2,5 ng/ml CEA, 10 ng/ml CEA und 20 ng/ml CEA) und des CEA-Kontrollserums (10,2 ng/ml CEA ± 1,0 ng/ml) zugemischt, je eine mit monoklonalem Maus-Anti-CEA-sensibilisierte Polystyrolkugel* (Ø = 6,5 mm) zugefügt und bei 45 °C während 4 Stunden inkubiert. Anschliessend werden die Polystyrolkugeln dreimal mit je 2-5 ml dest. Wasser gewaschen, in je 0,5 ml Substratpuffer für die Aktivitätsbestimmung der Peroxidase (0,1 mol/l Kaliumcitratpuffer vom pH 5,0 mit 6 mmol/l H$_2$O$_2$ und 40 mmol/l o-Phenylendiamin) transferiert und während 30 Minuten bei Raumtemperatur (22 °C) inkubiert. Zum Abstoppen der peroxidatischen Aktivität sowie zur Erhöhung der Farbintensität werden 2,0 ml 1 mol/l HCl zugemischt und innerhalb von 30 Minuten die Absorption bei der Wellenlänge 492 nm photometrisch gemessen. In der Tabelle I sind die Werte einer CEA-Bestimmung aufgeführt und einerseits mit den Werten verglichen, wie sie mit dem Radioimmunoassay von ROCHE erhalten wurden (Referenzwerte) und andererseits mit den Werten verglichen, wie sie nach einer modifizierten (Verwendung von Ziegen-anti-CEA-Peroxidasekonjugat in 0,2 M Phosphatpuffer anstele von TRIS-Puffer) Methode gemäss Clin. Chem. 26/12, 1718-1722 (1980) erhalten wurde.

*Die Herstellung des monoklonalen Maus-Anti-CEA erfolgt in Analogie zu der in Journal of Immunological Methods, 32 (1980) 297-304, beschriebenen Methode, wobei als Ausgangszellinie für die Fusion die Myelomlinie Sp 2/01-AG verwendet wird, welche unter der Nr. CRL 8006 bei ATCC am 25.5.79 hinterlegt und am 24.11.81 der Oeffentlichkeit uneingeschränkt zugänglich gemacht worden ist. Die Fusion erfolgt mit Milzzellen von Mäusen, die mit CEA immunisiert wurden. Die Immunisierung der Mäuse erfolgte in Analogie zu Tabelle I der erwähnten Publikation, wobei die ersten beiden Immunisierungen mit je 50 μg CEA erfolgten, die Immunisierungen 3 und 4 weggelassen wurden, Immunisierung 5 mit 50 μg CEA und die Immunisierungen 6-8 mit je 200 μg CEA erfolgten.

(Siehe Tabelle I Seite 4 f.)

0 072 902

Tabelle I

| Probematerial | $\Delta A_{492}$ nm/RT/30 Min. | | |
|---|---|---|---|
| CEA-Standard | | | |
| 0   ng/ml CEA | 0,105 | | |
| 2,5 ng/ml CEA | 0,330 | | |
| 5,0 ng/ml CEA | 0,490 | | |
| 10,0 ng/ml CEA | 0,755 | | |
| 20,0 ng/ml CEA | 1,220 | | |
| CEA-Kontrollserum | | | |
| 10,2 ng/ml CEA | 0,770 | | |
| Patienten-plasma | ROCHE-RIA-Test (CEA-Gehalt) | Modifizierte Methode nach Clin.Chem. (loc.cit.) (CEA-Gehalt) | Erfindungsge-mässes Verfahren (CEA-Gehalt) |
| No. Pool I | 0,8 ng/ml | 0,7 ng/ml | 0,7 ng/ml |
| No. Pool 2 | 1,4 ng/ml | 1,0 ng/ml | 1,0 ng/ml |
| No. Pool 3 | 2,3 ng/ml | 2,5 ng/ml | 2,4 ng/ml |
| No. 3155 | 25,0 ng/ml | 20,0 ng/ml | 22,0 ng/ml |
| No. 3157 | 3,3 ng/ml | 4,1 ng/ml | 3,7 ng/ml |
| No. 3368 | 7,1 ng/ml | 6,7 ng/ml | 6,9 ng/ml |
| No. 3395 | 14,8 ng/ml | 15,0 ng/ml | 14,7 ng/ml |
| No. 3401 | 9,0 ng/ml | 8,8 ng/ml | 9,2 ng/ml |
| No. 3410 | 23,0 ng/ml | 21,0 ng/ml | 24,0 ng/ml |
| No. 3419 | 4,2 ng/ml | 4,5 ng/ml | 4,4 ng/ml |
| No. 3416 | 4,1 ng/ml | 5,3 ng/ml | 5,2 ng/ml |
| No. 3646 | 5,5 ng/ml | 5,2 ng/ml | 5,3 ng/ml |
| No. 3657 | 5,2 ng/ml | 5,5 ng/ml | 5,5 ng/ml |
| No. 3679 | 5,7 ng/ml | 5,9 ng/ml | 6,0 ng/ml |

Werte unter 2,5 ng/ml CEA liegen im Normalbereich, während Werte über 2,5 ng/ml im pathologischen Bereich liegen. Aus der Tabelle I ist ersichtlich, dass die mit dem erfindungsgemässen Verfahren erhaltenen Werte gut mit denjenigen Werten korrelieren, die nach der modifizierten Methode in Clin. Chem. (loc. cit.) oder nach dem ROCHE-RIA-Test erhalten wurden.

Beispiel 2

Sensitivitätssteigerung der enzym-immunologischen CEA-Bestimmung mit einem monoklonalen Maus-anti-CEA und einem Ziegen-anti-CEA-Peroxidasekonjugat durch Zusatz von 0,8 mol/l Phosphationen

In die erforderliche Anzahl Teströhrchen (10 × 75 mm) werden je 0,5 ml Testlösung (2 g/l Rinderserumalbumin), 20 % Ziegenserum, 0,2 g/l 4-Amino-antipyrin und 0,2 µg/ml Ziegen-anti-CEA-Peroxidasekonjugat einmal in 0,2 mol/l Natriumphosphatpuffer und einmal in 0,8 mol/l Natriumphosphatpuffer vom pH-Wert 6,5) pipettiert, 0,050 ml der CEA-Standards (0 ng/ml CEA, 2,5 ng/ml CEA, 5,0 ng/ml CEA, 10,0 ng/ml CEA und 20,0 ng/ml CEA) und des CEA-Kontrollserums (10,2 ng/ml CEA ± 1,0 ng/ml) zugemischt, je eine mit monoklonalem Maus-CEA-Antikörper sensibilisierte Polystyrolkugel (Ø = 6,5 mm) zugefügt und bei 45 °C während 4 Stunden inkubiert. Anschliessend werden die Polystyrolku-

4

**0 072 902**

geln dreimal mit je 2-5 ml destilliertem Wasser gewaschen, in je 0,5 ml Substratpuffer für die Aktivitätsbestimmung der Peroxidase (0,1 mol/l Kaliumcitrat vom pH 5,0 mit 6 mmol/l $H_2O_2$ und 40 mmol/l o-Phenylendiamin) transferiert und während 30 Minuten bei Raumtemperatur (22 °C) inkubiert. Zum Abstoppen der peroxidatischen Aktivität sowie zur Erhöhung der Farbintensität werden 2,0 ml einer 1 mol/l HCl zugemischt und innerhalb von 30 Minuten die Absorption bei der Wellenlänge 492 nm photometrisch gemessen. In der Tabelle II sind die Werte aufgeführt, wie sie mit 0,2 mol/l und 0,8 mol/l Natriumphosphatpuffer erhalten wurden.

Tabelle II

| CEA Standards | $\Delta^A{}_{492}$ nm/RT/30 Min. | |
| --- | --- | --- |
| | mit 0,2 mol/l Phosphationen | mit 0,8 mol/l Phosphationen |
| 0 ng/ml CEA | 0,050 | 0,130 |
| 2,5 ng/ml CEA | 0,125 | 0,290 |
| 5,0 ng/ml CEA | 0,230 | 0,450 |
| 10,0 ng/ml CEA | 0,400 | 0,770 |
| 20,0 ng/ml CEA | 0,780 | 1,400 |
| CEA-Kontrollserum | | |
| 10,2 ng/ml CEA | 0,430 | 0,800 |

Aus dieser Tabelle II geht deutlich hervor, dass die Sensitivität unter Verwendung von 0,8 mol/l Phosphationen gegenüber 0,2 mol/l Phosphationen deutlich gesteigert wird.

Beispiel 3

Sensitivitätssteigerung der enzym-immunologischen CEA-Bestimmung mit einem monoklonalen Maus-anti-CEA und einem Ziegen-anti-CEA-Peroxidasekonjugat durch Zusatz von 0,4 mol/l Sulfationen

In die erforderliche Anzahl Teströhrchen (10 × 75 mm) werden je 0,5 ml Testlösung (2 g/l Rinderserumalbumin, 20 % Ziegenserum, 0,2 g/l 4-Amino-antipyrin und 0,2 µg/ml Ziegen-anti-CEA-Peroxidasekonjugat in 0,2 mol/l Natriumphosphatpuffer einmal ohne Sulfationen und einmal mit 0,4 mol/l Sulfationen ($Na_2SO_4$) vom pH-Wert 6,5) pipettiert, 0,050 ml der CEA-Standards (0 ng/ml CEA, 2,5 ng/ml CEA, 5,0 ng/ml CEA, 10,0 ng/ml CEA und 20,0 ng/ml CEA) und des CEA-Kontrollserums (10,2 ng/ml CEA ± 1,0 ng/ml) zugemischt, je eine mit monoklonalem Maus-CEA-Antikörper sensibilisierte Polystyrolkugel (∅ = 6,5 mm) zugefügt und bei 45 °C während 4 Stunden inkubiert. Anschliessend werden die Polystyrolkugeln dreimal mit je 2-5 ml destilliertem Wasser gewaschen, in je 0,5 ml Substratpuffer für die Aktivitätsbestimmung der Peroxidase (0,1 mol/l Kaliumcitrat vom pH 5,0 mit 6 mmol/l $H_2O_2$ und 40 mmol/l o-Phenylendiamin) transferiert und während 30 Minuten bei Raumtemperatur (22 °C) inkubiert. Zum Abstoppen der peroxidatischen Aktivität sowie zur Erhöhung der Farbintensität werden 2,0 ml einer 1 mol/l HCl zugemischt und innerhalb von 30 Minuten die Absorption bei der Wellenlänge 492 nm photometrisch gemessen. In der Tabelle III sind die Werte aufgeführt, wie sie mit 0,2 mol/l Natriumphosphatpuffer einmal ohne Sulfationen und einmal mit 0,4 mol/l Sulfationen erhalten wurden.

(Siehe Tabelle III Seite 6 f.)

5

# 0 072 902

Tabelle III

| CEA-Standards | $\Delta A_{492}$ nm/RT/30 Min. | |
|---|---|---|
| | ohne Sulfationen | mit 0,4 mol/l Sulfationen |
| 0    ng/ml CEA | 0,050 | 0,135 |
| 2,5  ng/ml CEA | 0,125 | 0,350 |
| 5,0  ng/ml CEA | 0,230 | 0,520 |
| 10,0 ng/ml CEA | 0,400 | 0,890 |
| 20,0 ng/ml CEA | 0,780 | 1,620 |
| CEA-Kontrollserum 10,2 ng/ml CEA | 0,430 | 0,930 |

Aus der Tabelle III geht deutlich hervor, dass durch die Anwesenheit der Sulfationen die Sensitivität erheblich gesteigert wird.

## Beispiel 4

Steigerung der Testempfindlichkeit durch erhöhte Phosphatkonzentration beim Einsatz von monoklonalem, biotinyliertem Anti-CEA und des Avidin-Peroxidase-Komplexes

In die erforderliche Anzahl Teströhrchen (10 × 75 mm) werden je 0,2 ml Testlösung (0,2 mol/l resp. 0,8 mol/l Natriumphosphatpuffer vom pH 6,5 mit 2 g/l Rinderserumalbumin, 20 % Ziegenserum, 0,1 g/l 4-Aminoantipyrin und 480 ng/ml monoklonales Maus Anti-CEA-(C-3)-Biotin/Avidin-Peroxidase) pipettiert. Zu dieser vorgegebenen Testlösung werden 0,050 ml der CEA-Standards (0 ng/ml CEA, 2,5 ng/ml CEA, 5,0 ng/ml CEA, 10 ng/ml CEA und 20 ng/ml CEA) zugemischt, je eine mit monoklonalem Maus Anti-CEA-(C-19)-sensibilisierte Polystyrolkugel (Ø = 6,5 mm) zugefügt und bei 37 °C während 16 Stunden inkubiert. Anschliessend werden die Polystyrolkugeln dreimal mit je 2-5 ml dest. Wasser gewaschen, in je 0,5 ml Substratpuffer (0,1 mol/l Kaliumcitrat vom pH 5,0 mit 6 mmol/l $H_2O_2$ und 40 mmol/l o-Phenylendiamin) für die Aktivitätsbestimmung der an die Kugel immunologisch gebundenen Peroxidase transferiert und während 30 Minuten bei 18-26 °C inkubiert. Zum Abstoppen der peroxidatischen Aktivität sowie zur Steigerung der Farbintensität werden 2,0 ml 1N HCl zugemischt und innerhalb von 30 Minuten die Extinktion bei der Wellenlänge 492 nm photometrisch gemessen. In der Tabelle IV sind die Werte für die CEA-Standards aufgeführt.

Tabelle IV

| CEA-Standard | $\Delta E_{492}$ nm / $20^{\circ}$C / 30 Min. | |
|---|---|---|
| | 0,2 mol/l Phosphatpuffer | 0,8 mol/l Phosphatpuffer |
| 0  ng/ml CEA | 0,080 / 0,080 | 0,090 / 0,095 |
| 2,5   "   " | 0,160 / 0,170 | 0,310 / 0,315 |
| 5,0   "   " | 0,230 / 0,235 | 0,450 / 0,460 |
| 10    "   " | 0,425 / 0,435 | 0,830 / 0,850 |
| 20    "   " | 0,810 / 0,825 | 1,700 / 1,650 |

6

Aus der Tabelle IV geht deutlich hervor, dass durch erhöhte Phosphatkonzentration die Sensitivität erheblich gesteigert wird.

Die in diesem Beispiel verwendeten monoklonalen Maus-Anti-CEA Antikörper erhält man in Analogie zu der in Journal of Immunological Methods, 32 (1980) 297-304 beschriebenen Methode, wobei jedoch als Ausgangszelllinie für die Fusion die erwähnte Myelomlinie Sp 2/01-AG verwendet wird.

Die Fusion erfolgt mit Milzzellen von Mäusen, die mit CEA immunisiert wurden. Die Immunisierung der Mäuse erfolgte in Analogie zu Tabelle 1 der erwähnten Publikation, wobei die ersten beiden Immunisierungen mit je 50 μg CEA erfolgten, die Immunisierungen 3 und 4 weggelassen wurden, Immunisierung 5 mit 50 μg CEA und die Immunisierungen 6-8 mit je 200 μg CEA erfolgten. Es werden zwei verschiedene, geeignete monoklonale Antikörper verwendet, die gegen unterschiedliche Epitope des CEA Antigens gerichtet sind und die als anti-CEA (C-3) bzw. anti-CEA (C-19) bezeichnet werden.

Die IgG-Fraktion von anti-CEA (C-3) Antikörpern wird über Nacht bei 2-8 °C gegen 0,1 mol/l Natriumbicarbonatlösung (pH 8,2-8,5) dialysiert und auf eine Proteinkonzentration von 1 mg/ml eingestellt. Dazu werden pro ml Proteinlösung 120 μl einer ganz frisch hergestellten Biotin-Succinimidester-Lösung (1 mg/ml in Dimethylsulfoxid) zugemischt und während 4 Stunden bei Zimmertemperatur inkubiert. Anschliessend wird das Biotin-Antikörper-Konjugat bei 2-8 °C gegen phosphatgepufferte, physiologische Kochsalzlösung mit 0,66 mg/l Merfen dialysiert. Als Proteinstabilisator werden 10 mg/ml Rinderserumalbumin beigegeben.

Für die Kopplung von Peroxidase an Avidin werden 5 mg Avidin in 2,5 ml einer 0,1 mol/l Natriumbicarbonatlösung (pH 9,5) gelöst und über Nacht bei 2-8 °C gegen die gleiche Lösung dialysiert. 10 mg Peroxidase aus Meerrettich werden in 3,0 ml dest. Wasser gelöst, während einer Stunde bei Raumtemperatur stehen gelassen, dann mit 0,5 ml einer wässrigen, frisch hergestellten 0,1 mol/l Natriumperjodatlösung vermischt und genau 20 Minuten bei Raumtemperatur inkubiert. Sodann wird die Peroxidase durch Ultrafiltration vom überschüssigen Natriumperjodat befreit und auf das ursprüngliche Volumen konzentriert. Nach der Ultrafiltration wird die Peroxidaselösung der Avidinlösung zugemischt, worauf man während 2 Stunden bei Raumtemperatur inkubiert. Zur Reduktion der Schiff'schen Basen und damit zur Stabilisierung der kovalenten Bindung werden der Avidin-Peroxidaselösung 0,5 ml einer frisch hergestellten, wässrigen, 0,1 mol/l Natriumborhydridlösung zugemischt, worauf man während mindestens 2 Stunden bei 2-8 °C inkubiert. Anschliessend wird das Avidin-Peroxidase-Konjugat gegen phosphatgepufferte, physiologische Kochsalzlösung mit 0,66 mg/l Merfen dialysiert und mit 10 mg/ml Rinderserumalbumin stabilisiert.

Zur Bildung des Komplexes werden 55 μl anti-CEA (C-3) Antikörper-Biotin-Konjugat mit 48 μl Avidin-Peroxidase-Konjugat gemischt und 15 Minuten bei Raumtemperatur inkubiert.

Für die erfindungsgemässe Verwendung wird der Komplex mit einer Lösung, die 0,20 mol/l bzw. 0,80 mol/l Natriumphosphatpuffer vom pH 6,5, 2 g/l Rinderserumalbumin, 20 % Ziegenserum und 0,1 g/l 4-Amino-antipyrin enthält, verdünnt.

**Patentansprüche**

1. Festphasen-Sandwichverfahren zur raschen Bestimmung von carcinoembryonalem Antigen (CEA) durch Inkubation der zu untersuchenden Probe mit einem ersten CEA-Antikörper, der an einen wasserunlöslichen Träger gebunden ist oder wird, und einem zweiten CEA-Antikörper, an den Peroxidase direkt oder über eine Biotin/Avidin-Brücke gebunden ist, Trennen von fester und flüssiger Phase, Messen der Peroxidase-Aktivität entweder in der festen oder in der flüssigen Phase als Ausmass des vorhandenen CEA, dadurch gekennzeichnet, dass die Inkubation in Gegenwart von 0,4 bis 1,0 mol/l Phosphationen, von 0,2-0,4 mol/l Sulfationen oder von 0,2-0,4 mol/l Tartrationen durchgeführt wird.

2. Verfahren nach Anspruch 1, dadurch gekennzeichnet, dass die Probe zuerst mit dem ersten CEA-Antikörper, der an einen wasserunlöslichen Träger gebunden ist oder wird, inkubiert wird und nach einem Waschschritt mit dem zweiten CEA-Antikörper, an den Peroxidase direkt oder über eine Biotin/Avidin-Brücke gebunden ist, inkubiert wird, wobei die zweite Inkubation in Gegenwart von 0,4 bis 1,0 mol/l Phosphationen, von 0,2-0,4 mol/l Sulfationen oder von 0,2-0,4 mol/l Tartrationen durchgeführt wird.

3. Verfahren nach Anspruch 1 oder 2, dadurch gekennzeichnet, dass bei einem Antikörper die Peroxidase direkt an den Antikörper gebunden ist.

4. Verfahren nach Anspruch 3, dadurch gekennzeichnet, dass die beiden CEA-Antikörper aus Antiseren verschiedener Tierspezies erhalten werden und dass bei einem Antikörper die Peroxidase direkt an den Antikörper gebunden ist.

5. Verfahren nach Anspruch 4, dadurch gekennzeichnet, dass man CEA-Antikörper von Ziegen und Meerschweinchen verwendet und dass beim Ziegen-Antikörper die Peroxidase direkt an den Antikörper gebunden ist.

6. Verfahren nach Anspruch 1 oder 2, dadurch gekennzeichnet, dass man zwei verschiedene monoklonale Maus-CEA-Antikörper verwendet und dass bei einem monoklonalen Maus-CEA-Antikörper die Peroxidase über eine Biotin/Avidin-Brücke an den Antikörper gebunden ist.

7. Verfahren nach Anspruch 3, dadurch gekennzeichnet, dass man einen CEA-Antikörper verwendet, der aus einem Antiserum eines Tieres erhalten wird, sowie einen monoklonalen Maus-CEA-Antikörper verwendet und dass beim Antikörper aus einem Antiserum die Peroxidase direkt an den Antikörper gebunden ist.

8. Verfahren nach Anspruch 7, dadurch gekennzeichnet, dass man als CEA-Antikörper Ziegen-CEA-Antikörper und monoklonale Maus-CEA-Antikörper verwendet und dass beim Ziegen-CEA-Antikörper die Peroxidase direkt an den Antikörper gebunden ist.

9. CEA-Antikörper, an die Peroxidase direkt oder über eine Biotin/Avidin-Brücke gekoppelt ist, in einer wässrigen Lösung vom pH 4-9, dadurch gekennzeichnet, dass die Lösung 0,4-1,0 mol/l Phosphationen, 0,2-0,4 mol/l Sulfationen oder 0,2-0,4 mol/l Tartrationen enthält.

10. CEA-Antikörper nach Anspruch 9, dadurch gekennzeichnet, dass die Peroxidase direkt an den Antikörper gekoppelt ist.

## Claims

1. A solid phase-sandwich method for the rapid determination of carcinoembryonic antigen (CEA) by incubation of the sample to be investigated with a first CEA antibody, which is or will be bound to a water-insoluble carrier, and a second CEA antibody, to which peroxidase is bound directly or via a biotin/avidin bridge, separation of the solid and liquid phase, measurement of the peroxidase activity either in the solid or in the liquid phase as the extent of CEA present, characterized by carrying out the incubation in the presence of 0.4 to 1.0 mol/l of phosphate ions, of 0.2-0.4 mol/l of sulphate ions or of 0.2-0.4 mol/l of tartrate ions.

2. A method according to claim 1, characterized in that the sample is incubated firstly with the first CEA antibody, which is or will be bound to a water-insoluble carrier, and after a washing step is incubated with the second CEA antibody, to wich peroxidase is bound directly or via a biotin/avidin bridge, whereby the second incubation is carried out in the presence of 0.4 to 1.0 mol/l of phosphate ions, of 0.2-0.4 mol/l of sulphate ions or of 0.2-0.4 mol/l of tartrate ions.

3. A method according to claim 1 or 2, characterized in that in the case of one antibody the peroxidase is bound directly to the antibody.

4. A method according to claim 3, characterized in that the two CEA antibodies are obtained from antisera of different animal species and in that in the case of one antibody the peroxidase is bound directly to the antibody.

5. A method according to claim 4, characterized in that CEA antibodies from goats and guinea pigs are used and in that in the case of the goat antibody the peroxidase is bound directly to the antibody.

6. A method according to claim 1 or 2, characterized in that two different monoclonal mouse-CEA antibodies are used and in that in the case of one monoclonal mouse-CEA antibody the peroxidase is bound to the antibody via a biotin/avidin bridge.

7. A method according to claim 3, characterized in that there is used a CEA antibody, which is obtained from an antiserum of an animal, as well as a monoclonal mouse-CEA antibody and in that in the case of the antibody from an antiserum the peroxidase is bound directly to the antibody.

8. A method according to claim 7, characterized in that goat-CEA antibody and monoclonal mouse-CEA antibody are used as the CEA antibodies and in that in the case of the goat-CEA antibody the peroxidase is bound directly to the antibody.

9. A CEA antibody, to which peroxidase is coupled directly or via a biotin/avidin bridge, in an aqueous solution of pH 4-9, characterized in that the solution contains 0.4-1.0 mol/l of phosphate ions, 0.2-0.4 mol/l of sulphate ions or 0.2-0.4 mol/l of tartrate ions.

10. A CEA antibody according to claim 9, characterized in that the peroxidase is coupled directly to the antibody.

## Revendications

1. Procédé sandwich en phase solide pour la détermination rapide d'un antigène carcinoembryonnaire (CEA) par incubation de l'échantillon à examiner avec un premier anticorps anti-CEA, qui est lié à un support insoluble dans l'eau ou qui le devient, et un second anticorps anti-CEA, sur lequel une peroxydase est liée directement ou par l'intermédiaire d'un pont biotine/avidine, de séparation de la phase solide et de la phase liquide, de mesure de l'activité de peroxydase en phase solide ou en phase liquide comme mesure du CEA présent, caractérisé en ce qu'on conduit l'incubation en présence de 0,4 à 1,0 mol/l d'ions phosphate, de 0,2-0,4 mol/l d'ions sulfate ou de 0,2-0,4 mol/l d'ions tartrate.

2. Procédé selon la revendication 1, caractérisé en ce qu'on fait tout d'abord incuber l'échantillon avec le premier anticorps anti-CEA, qui est ou devient lié à un support insoluble dans l'eau, et en ce qu'on le fait incuber après une étape de lavage avec le second anticorps anti-CEA, lié à la peroxydase directement ou par l'intermédiaire d'un pont biotine/avidine, la seconde incubation étant conduite en présence de 0,4 à 1,0 mol/l d'ions phosphate, de 0,2-0,4 mol/l d'ions sulfate ou de 0,2-0,4 mol/l d'ions tartrate.

3. Procédé selon les revendications 1 et 2, caractérisé en ce que dans un anticorps la peroxydase est liée directement à l'anticorps.

4. Procédé selon la revendication 3, caractérisé en ce qu'on obtient les deux anticorps anti-CEA à partir d'antiséra de différentes espèces animales et en ce que dans un anticorps la peroxydase est liée directement sur l'anticorps.

5. Procédé selon la revendication 4, caractérisé en ce qu'on utilise des anticorps anti-CEA de chèvre et de cobaye et en ce que dans l'anticorps de chèvre la peroxydase est liée directement sur l'anticorps.

6. Procédé selon les revendications 1 et 2, caractérisé en ce qu'on utilise deux anticorps monoclonaux anti-CEA de souris et en ce que dans un anticorps monoclonal anti-CEA de souris la peroxydase est liée à l'anticorps par l'intermédiaire d'un pont biotine/avidine.

7. Procédé selon la revendication 3, caractérisé en ce qu'on utilise un anticorps anti-CEA que l'on obtient à partir d'un antisérum d'un animal, ainsi qu'un anticorps monoclonal anti-CEA de souris, et en ce que dans l'anticorps obtenu à partir d'un antisérum la peroxydase est liée directement à l'anticorps.

8. Procédé selon la revendication 7, caractérisé en ce qu'on utilise comme anticorps anti-CEA un anticorps anti-CEA de chèvre et un anticorps monoclonal anti-CEA de souris, et en ce que dans l'anticorps anti-CEA de chèvre la peroxydase est liée directement aux anticorps.

9. Anticorps anti-CEA, auquel la peroxydase est couplée directement ou par l'intermédiaire d'un pont biotine/avidine, dans une solution aqueuse de pH 4-9, caractérisé en ce que la solution contient 0,4-1,0 mol/l d'ions phosphate, 0,2-0,4 mol/l d'ions sulfate ou 0,2-0,4 mol/l d'ions tartrate.

10. Anticorps anti-CEA selon la revendication 9, caractérisé en ce que la peroxydase est couplée directement aux anticorps.